# EUROPEAN PATENT APPLICATION

(11) **EP 4 763 166 A1**
(43) Date of publication of application: **24.06.2026**
(21) Application number: 24220824.7
(22) Date of filing: 17.12.2024
(51) Int. Cl.: A61F 13/15, A61F 13/49

(54) **ABSORBENT ARTICLE WITH IMPROVED ELASTICIZIED COMPONENT**

(71) Applicant: Ontex BV, 9255 Buggenhout (BE); Ontex Group NV, 9320 Erembodegem (BE)
(72) Inventor: Jansen, Bart, 2640 Mortsel (BE); Lambertz, Christina, 56566 Neuwied (DE); Alljasi, Majlinda, Aalst (BE)
(74) Representative: Saurat, Thibault

(57) **Abstract**

Absorbent article (10) comprising:
- a liquid permeable topsheet (20),
- a liquid impermeable backsheet (22), and
- an absorbent core (23) comprising absorbent material (24) positioned between said topsheet (20) and backsheet (22),
wherein the absorbent article (10) further comprises at least one component selected from front side panel (36), rear side panel (30), waist elastic (64), elastic waistband (66), gasketing elastic (38), leg elastic (41), edge elastic (72) characterized in that at least one of said at least one component comprises elastic material which is substantially free of N,N-dimethylacetamide and/or 1-ethylpyrrolidin-2-one.

## Description

### TECHNICAL FIELD

The disclosure relates to absorbent articles such as disposable absorbent articles, preferably selected from the group consisting of diapers (whether for baby or adults), pants (whether for baby or adults), pantiliners, briefs, sanitary napkins, and combinations thereof comprising an improved elasticized component.

### BACKGROUND

Disposable absorbent garments, or disposable absorbent articles, such as infant diapers or training pants, adult incontinence products and other such products typically are constructed with a liquid impervious outer backsheet, a liquid pervious body-contacting topsheet, and an absorbent core sandwiched between said topsheet and backsheet. Such disposable absorbent articles, usually comprise elasticized components.

It has been a constant objective in the recent years to manufacture absorbent articles containing the least possible amount of hazardous substances in other words, to minimize the presence of unwanted materials in order to provide a more pure end product to consumers desiring the same.

### SUMMARY

The object of the present invention pertains to an absorbent article comprising:
- a liquid permeable topsheet,
- a liquid impermeable backsheet, and
- an absorbent core comprising absorbent material positioned between said topsheet and backsheet,
wherein the absorbent article further comprises at least one component selected from front side panel, rear side panel, waist belt, elastic waistband, longitudinal and/or transversal cuffs, leg elastic, elasticized edge characterized in that at least one of said at least one component comprises elastic material which is substantially free of N,N-dimethylacetamide and/or 1-ethylpyrrolidin-2-one.

N,N-dimethylacetamide and/or 1-ethylpyrrolidin-2-one is a solvent commonly used in the process of manufacturing elastic material such as elastane, meaning that in traditional processes of making elastane there will always be traces of N,N-dimethylacetamide and/or 1-ethylpyrrolidin-2-one. This presence is unavoidable because elastane is made by dry spinning where polyurethane and a solvent such as N,N-dimethylacetamide and/or 1-ethylpyrrolidin-2-one are mixed and dried together thereby leaving traces of N,N-dimethylacetamide and/or 1-ethylpyrrolidin-2-one in the final product. These traces are undesired since N,N-dimethylacetamide and/or 1-ethylpyrrolidin-2-one are hazardous materials detrimental to the health of the wearer and caregiver. However, manufacturing processes such as melt spinning where the polyurethane is directly cooled down without the need for such hazardous solvents exist. It is therefore possible to use such materials to make an absorbent article that is safer and not containing unwanted materials.

According to an embodiment, said elastic material is selected from elastic strand, elastic film or elastic strip.

According to an embodiment, the elastic material comprises elastane or polyester.

According to an embodiment, the elastic material is a melt-spun material.

According to an embodiment, said at least one of said separable component is joined to the absorbent article by a rubber-based adhesive or a polyolefin adhesive or by mechanical bonding.

According to an embodiment, when said elastic material is selected from elastic strands or elastic threads said elastic strands or elastic threads have a fineness or thickness or a linear density between 300 dtex and 1500 dtex, preferably between 500 dtex and 1000 dtex, when said elastic material is selected from elastic strips or flat elastics said elastic strips or flat elastics have a fineness or thickness or a linear density between 1000 dtex and 5000 dtex, preferably between 2000 dtex and 4500 dtex, and when said elastic material is selected from elastic film said elastic film has a total basis weight comprised between 5 and 50 gsm, preferably between 8 and 30 gsm.

For sake of clarity, according to an embodiment, said elastic material is selected from elastic strands or elastic threads said elastic strands or elastic threads have a fineness or thickness or a linear density between 300 dtex and 1500 dtex, preferably between 500 dtex and 1000 dtex.

For sake of clarity, according to an embodiment, said elastic material is selected from elastic strips or flat elastics said elastic strips or flat elastics have a fineness or thickness or a linear density between 1000 dtex and 5000 dtex, preferably between 2000 dtex and 4500 dtex.

For sake of clarity, according to an embodiment, said elastic material is selected from elastic film said elastic film has a total basis weight comprised between 5 and 50 gsm, preferably between 8 and 30 gsm.

According to an embodiment, the absorbent core comprises biodegradable superabsorbent material (bioSAP), preferably carbohydrate fermentation-derived polymers and/or polysaccharides-derived polymers.

According to an embodiment, the absorbent material is wrapped in at least one core wrap substrate, said core wrap substrate comprising upper and lower layers that are joined together by one or more adhesives and/or by one or more mechanical bonds selected from the group consisting of ultrasonic bonds, thermal bonds, pressure bonds, and combinations thereof, preferably said layers are joined together at one or more attachment zones to form one or more channels, preferably a single channel, substantially free of absorbent material.

According to an embodiment, the absorbent core comprises cellulose fibers, said cellulose fibers being selected from unbleached pulp or totally chlorine free (TCF) or elemental chlorine free (ECF) cellulose fibers or any combination thereof.

The present disclosure pertains also to a method for manufacturing an absorbent article (10) according to any of the preceding claims, comprising the following steps:
a. providing a liquid permeable topsheet, a liquid impermeable backsheet, and an absorbent core comprising absorbent material, and at least one component selected from front side panel, rear side panel, waist belt, elastic waistband, longitudinal and/or transversal cuffs, leg elastic, elasticized edge said at least one component comprising elastic material which is substantially free of N,N-dimethylacetamide and/or 1-ethylpyrrolidin-2-one,
b. joining said topsheet, backsheet and absorbent core, and
c. joining said at least one component comprising elastic material which is substantially free of N,N-dimethylacetamide and/or 1-ethylpyrrolidin-2-one to at least one of said topsheet, backsheet and absorbent core.

According to an embodiment, wherein said elastic material is selected from elastic strand, elastic film or elastic strip.

According to an embodiment, the elastic material comprises elastane or polyester.

According to an embodiment, the elastic material is a melt-spun material.

According to an embodiment, said at least one of said separable component is joined to the absorbent article (10) by a rubber-based adhesive or a polyolefin adhesive or by mechanical bonding.

According to an embodiment, when said elastic material is selected from elastic strands or elastic threads said elastic strands or elastic threads have a fineness or thickness or a linear density between 300 dtex and 1500 dtex, preferably between 500 dtex and 1000 dtex, when said elastic material is selected from elastic strips or flat elastics said elastic strips or flat elastics have a fineness or thickness or a linear density between 1000 dtex and 5000 dtex, preferably between 2000 dtex and 4500 dtex, and when said elastic material is selected from elastic film said elastic film has a total basis weight comprised between 5 and 50 gsm, preferably between 8 and 30 gsm.

For sake of clarity, according to an embodiment, said elastic material is selected from elastic strands or elastic threads said elastic strands or elastic threads have a fineness or thickness or a linear density between 300 dtex and 1500 dtex, preferably between 500 dtex and 1000 dtex.

For sake of clarity, according to an embodiment, said elastic material is selected from elastic strips or flat elastics said elastic strips or flat elastics have a fineness or thickness or a linear density between 1000 dtex and 5000 dtex, preferably between 2000 dtex and 4500 dtex.

For sake of clarity, according to an embodiment, said elastic material is selected from elastic film said elastic film has a total basis weight comprised between 5 and 50 gsm, preferably between 8 and 30 gsm.

### BRIEF DESCRIPTION OF THE FIGURES

FIG. 1 illustrates a top view of an absorbent article according to an embodiment of the present disclosure;
FIG. 2 depicts a cross-section of an absorbent article according to an embodiment of the present disclosure;
FIG. 3 shows a view in perspective of an absorbent article according to an embodiment of the present disclosure;
FIG. 4 illustrates a top view of an absorbent article according to another embodiment;
FIG.5 depicts a cross-section of a portion of an absorbent article according to another embodiment;
FIG.6 shows a view in perspective of an absorbent article according to another embodiment;
FIG.7 illustrates a view in perspective of an absorbent article according to another embodiment;
FIG.8 represents a schematic drawings of a melt-spinning process;

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" or "weight percent" or "% wt.", here and throughout the description unless otherwise defined, refers to the relative weight of the respective compound based on the overall weight of a formulation, it can also refer to the relative weight of a respective component based on the overall weight of the absorbent article or of a larger element of said absorbent article. For example, if an elastic material such as a elastic thread is described as containing N,N-dimethylacetamide in a concentration lesser than 0.1 % wt., it is meant that the weight of N,N-dimethylaceteamide amounts to less than 0.1%wt. of the overall weight of the elastic thread.

The terms "biodegradable superabsorbent material" or "bioSAP" or "bipolar superabsorbent material" or "biocompostable superabsorbent material" or "superabsorbent material with a bio-based content" are equivalent terms and means a superabsorbent material that has the property to biodegrade, meaning that this superabsorbent material is capable of being broken down into innocuous products by the action of living elements such as microorganisms. Biodegradation, e.g., based on carbon to carbon dioxide conversion for solid materials (Sturm test), may be performed according to several methods such as ASTM D5210,ASTM D5988, ASTM D5511, ASTM D5864, ASTM D5271 and Iso 14593. Composability can be determined ASTM D6400, ISO 16929, 15014855. In addition, the term "biodegradable" can also mean a carbon-based material can be bacterially and/or enzymatically decomposed under conditions that are typically present in landfills. Such conditions usually include deep burial so that anaerobic bacterial and/or enzymatic activity typically will be the primary mode of decomposition. Such conditions typically do not include substantial non-bacterial hydrolysis but hydrolysis may occur to some extent. Typically, the biodegradable material is converted primarily, substantially, essentially or completely to ultimate decomposition products such as carbon dioxide, water and inorganic residue. The time needed for such full decomposition is usually substantial but bacterial and/or enzymatic decomposition will begin shortly after burial. "Bio-based content" refers to the amount of carbon from a renewable resource in a material as a percent of the mass of the total organic carbon in the material, as determined by ASTM D6866-10, method B. The methodology of ASTM D6866-10, method B, can be used to determine the bio-based content of any absorbent article or component thereof.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

"Laminate" refers to elements being joined in a layered arrangement.

The term "spunbond fibers" or "spunbond layer(s)" or "spunbond nonwoven(s)" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc.

The recitation of numerical ranges by endpoints includes all numbers and fractions subsumed within that range, as well as the recited endpoints unless otherwise stated.

"Carded web" or "carded layer(s)" or "carded nonwoven(s)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement. Carded thermobonded nonwoven thus refers to a carded nonwoven wherein the bonding is achieved by use of heat.

The term "topsheet" as used herein refers to a liquid permeable material sheet forming the inner cover of the absorbent article and which in use is placed in direct contact with the skin of the wearer. The topsheet is typically employed to help isolate the wearer's skin from liquids held in the absorbent structure. The topsheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The topsheet material may further be composed of two fibres, which may be bonded to each other in a bonding pattern. Further examples of topsheet materials are porous foams, apertured plastic films, laminates of nonwoven materials and apertured plastic films etc. The materials suited as topsheet materials should be soft and non-irritating to the skin and be readily penetrated by body fluid, e.g. urine or menstrual fluid. The inner coversheet may further be different in different parts of the absorbent article. The topsheet fabrics may be composed of a substantially hydrophobic material, and the hydrophobic material may optionally be treated with a surfactant or otherwise processed to impart a desired level of wettability and hydrophilicity.

"Adhesive" typically means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a rubber-based material; a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "backsheet" refers to a material forming the outer cover of the absorbent article. The backsheet prevents the exudates contained in the absorbent structure from wetting articles such as bedsheets and overgarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapour to escape from the absorbent material, while still preventing liquids from passing there through. Examples of breathable backsheet materials are porous polymeric films, nonwoven laminates of spunbond and meltblown layers and laminates of porous polymeric films and nonwoven materials.

"Acquisition and distribution layer" also known as "ADL" refers to a sub-layer which preferably is a nonwoven wicking layer under the topsheet of an absorbent product, which speeds up the transport and improves distribution of fluids throughout the absorbent core. The ADL is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the ADL is positioned between the topsheet and the retention portion.

As used herein, the term "transverse" or "lateral" refers to a line, axis, or direction which lies within the plane of the absorbent article and is generally perpendicular to the longitudinal direction.

"Thickness or caliper" herein are used interchangeably, and refer to the caliper typically measured as follows: at 0.5 kPa and at least five measurements are averaged. A typical testing device is a Thwing Albert ProGage system. The diameter of the foot is between 50 mm to 60 mm. The dwell time is 2 seconds for each measurement. The sample is to be stored at 23 + 2°C and at 50 + 2% relative humidity for 24 hours with no compression, then subjected to the fabric thickness measurement. The preference is to make measurements on the base substrate before modification, however, if this material is not available an alternative method can be used. For a structured substrate, the thickness of the first regions in between the second regions (displaced fiber regions) can be determined by using a electronic thickness gauge (for instance available from McMaster-Carr catalog as Mitutoyo No 547- 500). These electronic thickness gauges can have the tips changed to measure very small areas. For example, a blade shaped tip can be used that is 6.6mm long and 1 mm wide. Flat round tips can also be inserted that measure area down below 1.5mm in diameter. For measuring on the structured substrate, these tips are to be inserted between the structured regions to measure the as-produced fabric thickness. The pressure used in the measurement technique cannot be carefully controlled using this technique, with the applied pressure being generally higher than 0.5kPa.

As used herein, the term "cellulosic" or "cellulose" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

By "substantially", it is meant at least the majority of the structure referred to.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "consisting essentially of' does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

As used herein, the "body-facing" or "bodyside" or "skin-facing" surface means that surface of the article or component which is intended to be disposed toward or placed adjacent to the body (e.g. the face) of the wearer during ordinary use, while the "outward", "outward-facing" surface is on the opposite side, and is intended to be disposed to face away from the wearer's body during ordinary use.

As used herein, "rear" and "back" have the same meaning and are interchangeable, said rear and back are usually in reference to the longitudinal axis or direction and correspond to the direction opposite to the front, hence rear or back region are in opposition to the front region. This applies for example to the back region or to the back waist belt.

The expression "elastic material which is substantially free of N, N-dimethylacetamide and/or 1-ethylpyrrolidin-2-one" it is meant that said elastic material contain these substance(s) in a concentration lesser than 0.1 % wt.. Preferably, said elastic material contains less than 10 PPM (parts per million), preferably less than 1 PPM.

Embodiments of the articles and processes according to the disclosure will now be described. It is understood that technical features described in one or more embodiments maybe combined with one or more other embodiments without departing from the intention of the disclosure and without generalization therefrom.

### General description of an absorbent article

According to the present disclosure, "absorbent article(s)" refers to a device(s) that absorbs and contains liquid, and more specifically, refers to device(s) that is placed against or in proximity to the body of the wearer to absorb and contains the various exudates discharged from the body such as urine, menstrual blood, fecal matter, *etc.* Absorbent articles comprise a longitudinal axis defining a length, a transversal axis defining a width, said transversal axis being perpendicular to said longitudinal axis as well as a vertical axis defining a thickness. The longitudinal axis is hereby conventionally chosen in the front-to-rear, or front-to-back, direction of the article when referring to the article being worn, and the transversal axis is conventionally chosen in the left-to-right direction of the article when referring to the article being worn.

Disposable or reusable absorbent articles 10 include a liquid pervious topsheet 20, a liquid impermeable backsheet 22 joined to the topsheet 20, and an absorbent core 23 positioned and held between the topsheet 20 and the backsheet 22. The topsheet 20 is operatively permeable to the liquids that are intended to be held or stored by the absorbent article, and the backsheet 22 is substantially liquid impervious or otherwise operatively impermeable to the intended liquids. The absorbent article 10 may also include other components, such as acquisition distribution layers 25, liquid intake layers, liquid distribution layers, transfer layers, barrier layers, wrapping layers and the like, as well as combinations thereof. Disposable or reusable absorbent articles 10 and the components thereof can operate to provide a body-facing surface and a garment-facing surface.

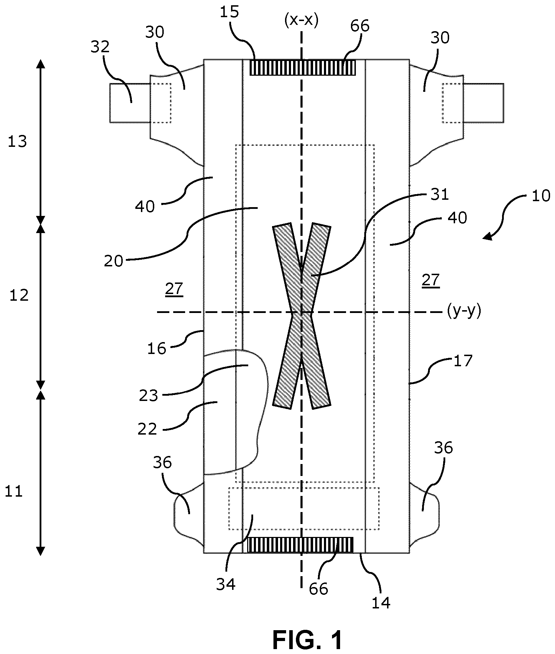

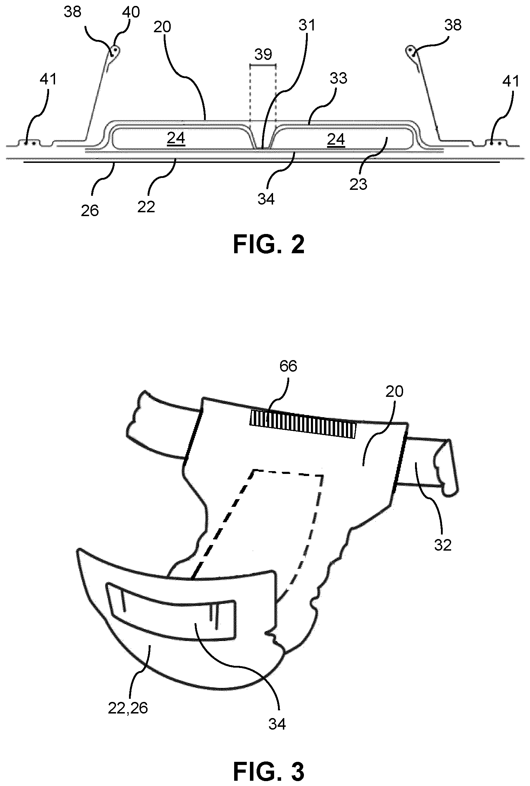

An example of absorbent article 10 according to the present disclosure is shown in FIG. 1, FIG. 2 and FIG. 3, in the form of a diaper with closing tapes. FIG. 1 is a plan view of the diaper 10 with the topsheet 20 facing the viewer in a flat configuration, i.e. said diaper 10 is in a laid-out state with no elastic contraction. FIG. 2 is a cross-sectional view along the transversal axis (y-y) of the diaper 10 of FIG. 1 at the intersection between the longitudinal (x-x) and transversal axis (y-y). FIG. 3 is a perspective view of the diaper 10 with elastic contraction. The absorbent article 10 as shown in FIG. 1 to FIG. 3 is shown for illustration purposes only as the present disclosure may be used for making a wide variety of diapers, including adult incontinence products, pants, or other absorbent articles, such as sanitary napkins and absorbent pads, for example.

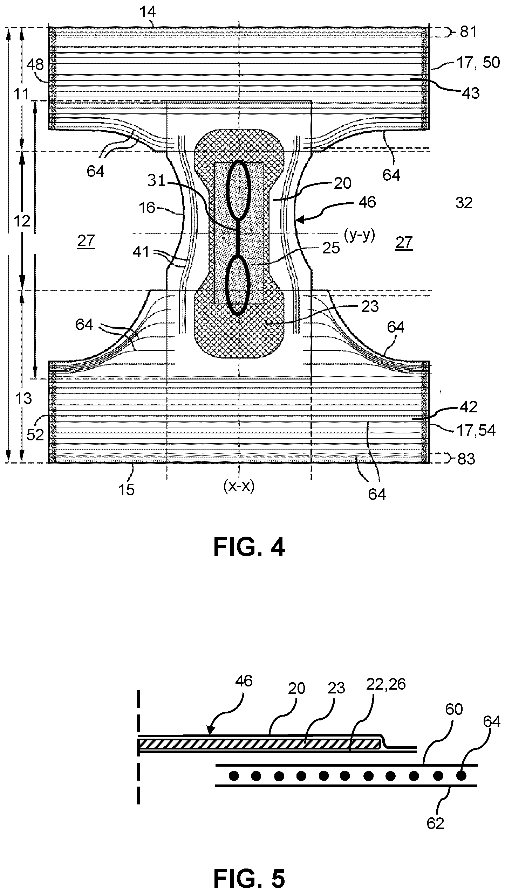

Another example of absorbent article 10 according to the present disclosure is shown in FIGS. 4, 5 and 6 in the form of a pant having permanent or refastenable side seams.

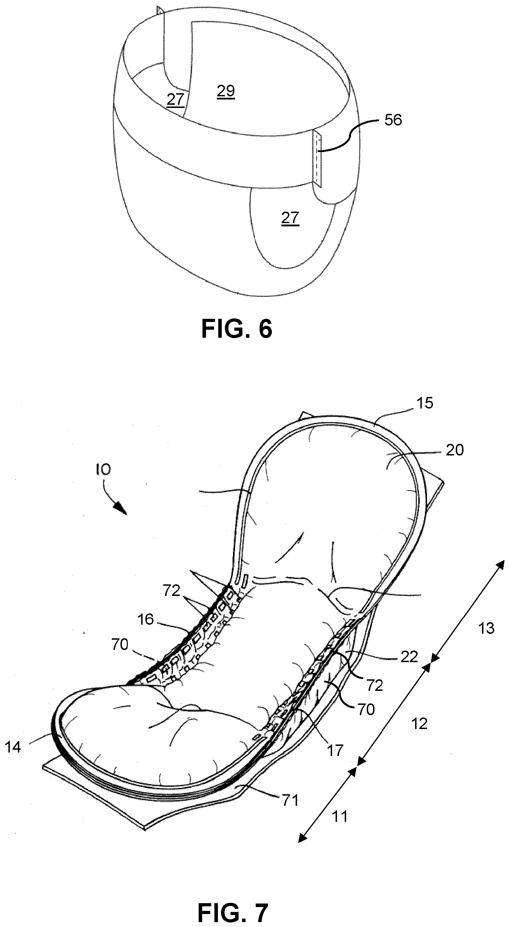

Here, FIG. 4 is a plan view of the pant 10 with the topsheet 20 facing the viewer in a flat laid-out state with no elastic contraction. FIG. 5 is a cross-sectional view along the transversal axis (y-y) of the pant 10 of FIG. 4. FIG. 6 is a perspective view of the pant 10 in a fastened configuration, meaning as intended to be used configuration. The absorbent article 10 as shown in FIG. 4 to FIG. 6 is also shown for illustration purposes only as the present disclosure may be used for making a wide variety of diapers, including adult incontinence products, pants, or other absorbent articles, such as sanitary napkins and absorbent pads, for example.

Another example of absorbent article 10 according to the present disclosure is shown in FIG. 7 in the form of an absorbent pad having elasticized edges. FIG. 7 is a perspective view of the pad 10 in an unfolded configuration, meaning in a as intended to be used configuration. The absorbent article 10 as shown in FIG. 7 is also shown for illustration purposes only as the present disclosure may be used for making a wide variety of diapers, including adult incontinence products, pants, or other absorbent articles, such as sanitary napkins and absorbent pads, for example.

Elements of FIG. 1 to FIG. 7 having the same reference number correspond to the same element. The absorbent article 10 has a central transversal (or lateral) axis (y-y) and a central longitudinal axis (x-x). The central transversal axis (y-y) extends perpendicular to the central longitudinal axis (x-x).

According to the present disclosure, the absorbent article 10 selected from, and not limited to, baby diapers, adult incontinence pants, baby pants, menstrual pants, absorbent pads and other absorbent articles such as sanitary napkins, comprises a front region 11, a crotch region 12, and a rear or back region 13, the crotch region 12 extending between the front region 11 and the rear region 13. The front region 11, the crotch region 12, and the rear region 13 can each be about one-third of the length of the absorbent article 10. The absorbent article 10 comprises a front-end edge 14, a rear-end edge 15 opposite to the front-end edge 14, and longitudinally extending, transversely opposed side edges 16,17 arranged between the front-end and rear-end edges 14,15, for example left-side and right-side edges 16,17. The crotch region 12, the front region 11 and the rear region 13 define leg openings 27 of the diaper-type or pant-type absorbent article 10 in a fastened configuration.

As explained hereabove, the absorbent article 10 comprises a liquid permeable topsheet 20, a liquid impermeable backsheet 22, and an absorbent core 23 comprising absorbent material 24 positioned at least partially between the topsheet 20 and the backsheet 22, preferably the absorbent core 23 is entirely enveloped or encased between the topsheet 20 and backsheet 22. The topsheet 20 and backsheet 22 are joined directly or indirectly. The absorbent article 10 can optionally further comprise an acquisition distribution layer 25 positioned between the topsheet 20 and the absorbent core 23. An outer cover material 26, such as a nonwoven material, may cover a garment-facing side of the backsheet 22.

The absorbent material 24 can be contained within at least one core wrap substrate(s) 33,34 enclosing said absorbent material 24 therein. The core wrap substrate 33,34 may be a single sheet that is folded onto itself to contain the absorbent material 24 therein or may comprise two distinct sheets that are bonded to each other at least at the peripheral edges thereof to sandwich the absorbent material 24 therein. Several core wrap constructions are possible such as G-fold, C-fold or sandwich wraps. The core wrap substrates 33,34 herein are typically composed of a nonwoven layer or nonwoven laminates comprising spunbond nonwoven, meltblown nonwoven and combinations thereof or tissue. The absorbent article 10 may comprise at least one channel 31. The term "channel(s)" as used herein means fluid distribution means within the absorbent core 23 and/or acquisition distribution layer 25 that are adapted to favour exudate flow there along and generally include structures that are substantially free of absorbent material instead of comprising compacted absorbent material. The channels 31 as disclosed herein can be formed by joining the core wrap substrates 33,34. Channels 31 preferably comprise recessed regions forming visible conduits or passages typically extending along the longitudinal axis of the core and having a depth in a direction perpendicular to said longitudinal axis. By "visible" it is herein intended clearly visible by naked eye. Typically the channels have a width 39 generally greater than 1 mm, preferably from 5 mm to 50 mm, more preferably from 6 mm to 40 mm, more preferably from 8 mm to 30 mm, even more preferably from greater than 8 mm to less than 25 mm. The one or more channels 31 are arranged within the inner periphery of the absorbent core 23, i.e. the inner, or interior, space defined by the periphery of the absorbent core 23, such that each of the channel(s) 31 is bounded, or surrounded, by absorbent material 24. Additionally, the length of the channels 31 may be from 10 % to 95 % of the length of the absorbent core so that again the one or more channels are bounded by absorbent material. Typically the basis weight of the core wrap substrate is from 5 gsm to 50 gsm, preferably from 6 gsm to 35 gsm, more preferably from 8 gsm to 30 gsm. The absorbent material 24 may be contained within the absorbent article 10 by the topsheet 20 and backsheet 22 only without the presence of a core wrap substrate. Since the object of the present disclosure pertains to less hazardous absorbent articles, according to the present disclosure, the core wrap substrate 33,34 can be made of environmentally friendly material, preferably biodegradable and/or compostable material, selected from and not limited to polylactic acid (PLA), viscose, polyhydroxyalcanoates (PHA), bio-derived polyethylene, (bioPE), low density polyethylene (LDPE), poly-3-hydroxybutyrate (PHB) or polycaprolactone (PCL).

As illustrated in FIG. 1, the absorbent article 10 may comprise rear side panels 30 in the rear region 13, also called back side panels. The rear side panels 30 comprise fasteners 32; also called closing tapes 32, and extend from the rear region 13 of the absorbent article 10 and can be attached, using the fasteners 32, to the landing zone 34 or landing zone material 34 arranged on a garment-facing portion of the front region 12 of the absorbent article 10. The fasteners and landing zone material comprise complementary attachment means such as hooks and loops, adhesive and a receiving surface, etc. The absorbent article 10 may also comprise front side panels 36 in the front waist region 11. The front side panels 36 are optional but are preferable as they improve the snug fit and overall comfort of the wearer. Front or rear side panels are commonly called ears, i.e. the front ears and the rear ears and correspond to a separate component in the form of a panel that is attached to the chassis meaning the front and/or rear side panels can be attached to the topsheet, backsheet or absorbent core or any combination thereof. For example the side panels 30,36 can be glued to the backsheet or the side panels 30,36 can be fixedly sandwiched between the topsheet and backsheet or the side panels 30,36 can be welded to the topsheet or the side panels 30,36can be attached to another component such as the barrier leg cuffs. For example, the side panels 30,36 can be glued between the barrier leg cuffs and the backsheet. The front and/or rear side panels are further defined hereunder.

As illustrated in FIG. 4, the absorbent article 10 may comprise a front waist belt 43 in the front region 11 and a back waist belt 42 in the rear region 13 and an absorbent insert 46 comprising a liquid permeable topsheet 20, a liquid impermeable backsheet 22, and an absorbent core 23 comprising absorbent material positioned at least partially between the topsheet and the backsheet. The absorbent insert 46 may be joined to a wearer-facing surface of the front and back waist belts 43, 42 or to a garment-facing surface of the front and back waist belts 43,42. Side edges 48 and 50 of the front waist belt 43 may be joined to side edges 52 and 54, respectively, of the rear waist belt 42 to form two side seams 56. As shown in FIG. 6, the side seams 56 may be any suitable seams known to those of skill in the art, such as butt seams or overlap seams, for example. When the side seams 56 are permanently formed or refastenably closed, the absorbent article 10 in the form of a pant has two leg openings 27 and a waist opening circumference 29. The side seams 56 may be permanently joined using adhesives or bonds such as ultrasonic bonding or mechanical bonding, for example, or may be refastenably closed using hook and loop fasteners, for example.

As shown in FIG. 7, the absorbent article 10 may comprise a layer of adhesive on the garment side of the backsheet 22 and comprise a release paper, or release liner 71 corresponding to a removable peel strip that may be removed to expose said adhesive to fasten the absorbent article 10 to an undergarment. For example, the release paper 71 can be a removable paper layer that is coated with peeling agent, such as silicone and arranged to cover or protect a layer of adhesive arranged on the backsheet 22.

According to the present disclosure, the absorbent article 10, selected from, and not limited to, baby diapers, adult incontinence pants, baby pants, menstrual pants, absorbent pads and other absorbent articles such as sanitary napkins, comprises at least one component comprising elastic material. This component is a separable component, meaning it is assembled, or joined, to at least one element of the absorbent article 10 and does not form a continuity of matter with said element of the absorbent article 10. In other words, these components comprising elastic material are not monolithic with the rest of the absorbent article 10.

The component comprising elastic material is selected from and not limited to barrier leg cuff(s), gasketing elastic(s), leg elastic(s) also known as gasketing cuff(s), elastic waistband(s), waist elastic(s), front and/or back side panel(s), elasticized edge(s). These components are further described hereunder. For example, the absorbent article 10 may comprises one or more barrier leg cuffs 40 with elastic strands 38, one or more leg elastics 41, one or more elastic waistbands and/or one or more elastic side panel. Each of these component can be joined for example via heat sealing, ultrasonic bonding, thermal bonding, by adhesives, stitching, or the like to another element of the absorbent article 10 such as the topsheet 20, the backsheet 22, the outer cover 26, the ADL 25, the absorbent core 23 or the overall absorbent insert 46.

According to the present disclosure, the component comprising elastic material is substantially free of N,N-dimethylacetamide and/or 1-ethylpyrrolidin-2-one. These compounds are solvents commonly used in the process of manufacturing elastic material such as elastane. Meaning that the traditional processes of making elastane imply the presence of N,N-dimethylacetamide and/or 1-ethylpyrrolidin-2-one in the final elastane compound. This presence is due to the fact that elastane is made by dry spinning where polyurethane and a solvent such as N,N-dimethylacetamide and/or 1-ethylpyrrolidin-2-one are mixed and dried together thereby leaving residue of N,N-dimethylacetamide and/or 1-ethylpyrrolidin-2-one in the final product, meaning the elastic material. This is undesired since N,N-dimethylacetamide and/or 1-ethylpyrrolidin-2-one are hazardous materials detrimental to the health of the wearer and caregiver.

According to the present disclosure, the component comprising elastic material is substantially free of N,N-dimethylacetamide and/or 1-ethylpyrrolidin-2-one by using N,N-dimethylacetamide-free and/or 1-ethylpyrrolidin-2-one-free elastane. Said N,N-dimethylacetamide-free and/or 1-ethylpyrrolidin-2-one-free elastane is made with a process such as melt spinning where the polyurethane is directly cooled down without the need for such hazardous solvent.

According to the present disclosure, the component comprising elastic material comprises a melt-spun elastic material. Simply put, the elastic material is a melt-spun material. Melt-spun elastic materials have the benefit of having little to no odor, namely since melt-spun elastic materials are substantially free of acetic acid.

The substantially free of N,N-dimethylacetamide and/or 1-ethylpyrrolidin-2-one elastic material, for example the N,N-dimethylacetamide-free elastane and/or the 1-ethylpyrrolidin-2-one-free elastane can be made under various forms such as elastic strand(s), elastic film(s) or elastic strip(s). In other words, the elastic material is selected from elastic strand(s) or elastic thread(s), elastic film(s) or elastic strip(s) or flat elastic(s).

As explained hereabove, said elastic material comprises elastane. In embodiments where the elastic material is selected from elastic strands or elastic threads said elastic strands or elastic threads have a fineness or thickness or a linear density between 300 dtex and 1500 dtex, preferably between 500 dtex and 1000 dtex, in embodiments where the elastic material is selected from elastic strips or flat elastics said elastic strips or flat elastics have a fineness or thickness or a linear density between 1000 dtex and 5000 dtex, preferably between 2000 dtex and 4500 dtex and in embodiments where the elastic material is selected from elastic film said elastic film has a total basis weight comprised between 5 and 50 gsm, preferably between 8 and 30 gsm.

According to the present disclosure, said at least one component comprising said elastic material is connected to the absorbent article by a rubber-based adhesive. Such rubber-based adhesive has the benefit of being more natural or sustainable than conventional petroleum-based adhesive. According to another embodiment, said at least one component comprising said elastic material is connected to the absorbent article, meaning to the rest of the absorbent article, by polyolefin-based adhesive or by mechanical bonding techniques such as ultrasonic welding.

The component comprising elastic material is selected from and not limited to longitudinal and/or transversal cuff(s) or barrier leg cuffs, leg elastics also known as gasketing elastics, waistband(s), waist elastic(s), front and/or rear side panel(s) or elasticized edge(s) or combination thereof. These component are now further described hereunder.

### Longitudinal and/or transversal cuffs 40 and gasketing elastics 38

The absorbent article 10 may comprise one or more pairs of longitudinal and/or transversal cuffs 40 comprising one or more gasketing elastics 38. The longitudinal and/or transversal cuffs 40 can be in the form of barrier leg cuffs or of an exudates containment pocket or poo pocket or a combination thereof. The absorbent article 10 as shown in FIG. 2 comprises a pair of barrier leg cuffs 40 each comprising a pair of gasketing elastics 38. Each barrier leg cuff 40 may be formed by a piece of material which is bonded to the absorbent article 10 so it can extend upwards from a wearer-facing surface of the absorbent article 10 and provide improved containment of body exudates approximately at the junction of the torso and legs of the wearer. Useful materials include, but are not limited to, spunbond-meltblown-spunbond (SMS) nonwoven webs. Such webs can comprise non-phthalate catalyst polypropylene fibers, and/or plant-based fibers. The longitudinal and/or transversal cuffs 40 are delimited by a proximal edge joined directly or indirectly to the topsheet 20 and/or the backsheet 22 and a free terminal edge, which is intended to contact and form a seal with the wearer's skin. The longitudinal cuffs 40 may extend at least partially between the front-end edge 14 and the rear-end edge 15 of the absorbent article 10 on opposite sides of the central longitudinal axis (x-x) and is present at least in the crotch region 12 and may also be present in the front and rear regions 11;13. The proximal edge is proximal to the topsheet 20 and/or backsheet 22 whereas the free terminal edge is distal to the topsheet 20 and/or backsheet 22 namely with respect to the vertical direction. The barrier leg cuffs 40 each comprise one or more gasketing elastic(s) 38 such as elastic strands or elastic strips, near or at the free terminal edge. For example, at its' free terminal edge the barrier leg cuff 40 can be folded and bonded onto itself with the gasketing elastic(s) 38 being enclosed within said fold. The gasketing elastic(s) 38 can be separated or removed from the absorbent article 10 by unfolding said fold and removing said elastic(s) 38, eventually by applying an adhesive remover if the gasketing elastic(s) 38 were glued into said fold. These gasketing elastic(s) 38 lead the barrier leg cuffs 40 to form a seal around the legs and torso of a wearer thanks to the elastic tension or elastic contraction of the gasketing elastics 38. The transversal cuff(s) 40 may extend partially between the side edges and preferably covers a portion of the absorbent core 23 so that the exudates can be absorbed by the absorbent core 23. The transversal cuff(s) acts as a containment element extending transversally in the front and/or back region 11,13 preferably at the edge(s) of the absorbent core 23. The transversal cuff(s) 40 also comprises a gasketing elastic 38 so that the transversal cuff 40 can extend upward and be lifted from the absorbent core 23 thereby acting as a containment barrier for the exudates.

According to the present disclosure, the gasketing elastic(s) 38 comprise elastic material which is substantially free of N,N-dimethylacetamide and/or 1-ethylpyrrolidin-2-one. According to an embodiment, the gasketing elastic(s) 38 comprises melt-spun elastic material, preferably, the gasketing elastic(s) 38 is made of melt-spun elastic material, more preferably, the gasketing elastic(s) 38 consists essentially of melt-spun elastic material. As explained melt-spun elastic materials have the benefit of being substantially free of N,N-dimethylacetamide and/or 1-ethylpyrrolidin-2-one, meaning that these melt-spun elastic materials comprise no or insignificant traces of these hazardous compounds, meaning in such insignificant amounts that it cannot be considered as harmful to the health. According to an embodiment, the gasketing elastic(s) 38 comprises melt-spun elastane. The gasketing elastics 38 comprising melt-spun elastic material in form of elastic strands or elastic threads said elastic strands or elastic threads comprising, preferably consisting of, elastane and having a fineness or thickness or a linear density between 300 dtex and 1500 dtex, preferably between 500 dtex and 1000 dtex.

Elastane is a polyether-polyurea copolymer. It is a derivative of polyurethane meaning elastane is made up of polyglycol combined with diisocyanate, and contains at least 85 % wt. of polyurethane, meaning the elastane comprises at least 85% wt. of polyurethane. For example an elastic thread made of elastane contains at least 85 % wt. of polyurethane.

### Leg Elastics 41

Referring to FIG. 2 or FIG. 4, the absorbent article 10 may comprise one or more pair of leg elastics 41, here two pairs of leg elastics 41 (FIG. 2) or three pairs or leg elastics 41 (FIG. 4). The leg elastics 41 are positioned laterally outboard of gasketing elastics 38. Each leg elastic 41 may be formed by a piece of material which is bonded to the absorbent article 10. The leg elastic(s) 41 extends at least partially between the front-end edge 14 and the rear-end edge 15. The leg elastic(s) 41 essentially leads portions of the absorbent article 10 proximate to the side edges 16,17 to help form a seal around the legs of the wearer. The leg elastic(s) 41 extends at least within the crotch region 12 and may extend at least partially within the front and rear regions 11,13. For example, the leg elastics 41 may be separated from the absorbent article 10 by removing the barrier leg cuffs or the topsheet or backsheet, eventually by force if the leg elastics 41 have been mechanically bonded onto the topsheet or backsheet. According to the present disclosure, the leg elastic(s) 41 comprises elastic material which is substantially free of N,N-dimethylacetamide and/or 1-ethylpyrrolidin-2-one. According to an embodiment, the leg elastic(s) 41 comprises melt-spun elastic material, preferably, the leg elastic(s) 41 is made of melt-spun elastic material, more preferably, the leg elastic(s) 41 consists essentially of melt-spun elastic material. According to an embodiment, the leg elastic(s) 41 preferably comprises melt-spun elastane. The leg elastic(s) 41 comprising melt-spun elastic material have a in form of elastic strands or elastic threads said elastic strands or elastic threads comprising, preferably consisting of, elastane and having a fineness or thickness or a linear density between 300 dtex and 1500 dtex, preferably between 500 dtex and 1000 dtex..

### Front and back waist belts 43, 42 and waist elastics 64

Referring to FIG. 4 and FIG. 5, the front and back waist belts 43,42 may each comprise an inner waist belt layer 60 and an outer waist belt layer 62 and an elastic component, namely at least one waist elastic(s) 64 disposed at least partially between the inner and outer waist belt layers 60,62. The waist elastic(s) 64 may be in the form of a plurality of elastic strands as illustrated in FIG. 4 or FIG.5, of a plurality of elastic strips or of one or more elastic film(s) or a combination thereof. The elastic film may be relaxed (including being cut) to reduce elastic strain over the absorbent insert 46 or, may alternatively, run continuously across the absorbent core 30. The elastic strands or threads 64 may extend continuously across the front and back waist belts 43,42 and/or the elastic strands or threads 64 may extend partially across the front and/or back waist belts 43,42, for example on both sides of the absorbent insert 46 but not under said absorbent insert as illustrated in FIG. 4. The waist elastic(s) 64 may have uniform or variable spacing therebetween in any portion of the waist belts. The waist elastic(s) 64 may also be pre-strained by the same amount or different amounts meaning the waist elastic(s) may be pre-strained to the same or different stretch ratios. The front and/or rear waist belts 43,42 may have one or more elastic component free zones, meaning a gap between said elastic components. In other instances, at least some of the elastic components 64 may extend continuously across the waist belt 43,42. The inner and outer waist belt layers 60,62 may be joined using adhesives, heat bonds, pressure bonds or thermoplastic bonds.

The front and rear waist belts 43,42 may be continuous, meaning having at least one layer that is continuous from one waist belt side edge to the other edge (from 48 to 50 and from 52 to 54). Alternatively, the front and rear waist belts 43,42 may be discontinuous from waist belt side edge to waist belt side edge, such that they are discrete.

There may be different arrangement of the waist elastics 64, the waist elastics 64 can be arranged in an area 81,83 proximal to the front-end and rear-end edges 14, 15, the waist elastics 64 are extending at a separation from each other which is at least 50% smaller than the separation of the waist elastics 64 in the remaining area of said front region 11 and/or the rear region 12. This ensures a better fitting of the absorbent article around the wearer's waist. For example, said area 81,83 extends from 10 mm to 30 mm, for example between 12 mm and 25 mm. In said area 81,83, the waist elastics 64 may have a minimum separation of 0.5 mm to 5 mm, preferably of 1 mm to 4 mm. By separation, it is meant the separation of a directly neighbouring elastic.

The waist elastics 64 may extend rectilinearly from one side edge to another side edge 48,50,52,54 and/or the waist elastics 64 may extend starting from a side edge 48,50,52,54 towards the central longitudinal axis x-x of the absorbent article 10 in a curved shape and fan out with increasing separation from each other.

The front and rear outer waist belt layer 62 may be separated from each other, such that the layers are discrete or, alternatively, these layers may be continuous, such that a layer runs continuously from the front-end edge 14 to the rear-end edge 15. This may also be true for the front and rear inner waist belt layers 60 that is, they may also be longitudinally discrete or continuous.

According to the present disclosure, the waist elastic(s) 64 comprises elastic material which is substantially free of N,N-dimethylacetamide and/or 1-ethylpyrrolidin-2-one. According to an embodiment, the waist elastic(s) 64 comprises melt-spun elastic material, preferably, the waist elastic(s) 64 is made of melt-spun elastic material, more preferably, the waist elastic(s) 64 consists essentially of melt-spun elastic material. According to an embodiment, the waist elastic(s) 64 preferably comprises melt-spun elastane. The waist elastic(s) 64 comprising melt-spun elastic material in form of elastic strands or elastic threads said elastic strands or elastic threads comprises, preferably consisting of, elastane and has a fineness or thickness or a linear density between 300 dtex and 1500 dtex, preferably between 500 dtex and 1000 dtex. The waist elastic(s) 64 comprising melt-spun elastic material in form of elastic strips or flat elastics said elastic strips or flat elastics have a fineness or thickness or a linear density between 1000 dtex and 5000 dtex, preferably between 2000 dtex and 4500 dtex. The waist elastic(s) 64 comprising melt-spun elastic material in form of elastic film said elastic film has a total basis weight comprised between 5 and 50 gsm, preferably between 8 and 30 gsm.

### Elastic Waistband 66

Referring to FIG. 1 or FIG. 3, the absorbent article 10 may comprise one or more elastic waistbands 66. The elastic waistband(s) 66 may be positioned on the garment-facing surface or the wearer-facing surface. As an example, a first elastic waistband 66 may be present in the front region 11 near the front-end edge 14 and a second elastic waistband 66 may be present in the rear region 13 near the rear-end edge 15. The elastic waistband(s) 66 aid in sealing the absorbent article 10 around a waist of a wearer and at least inhibiting bodily exudates from escaping the absorbent article 10 through the waist opening circumference 29. In some instances, an elastic waistband 66 may fully surround the waist opening circumference of an absorbent article. According to the present disclosure, the elastic waistband(s) 66 comprises elastic material which is substantially free of N,N-dimethylacetamide and/or 1-ethylpyrrolidin-2-one. According to an embodiment, the elastic waistband(s) 66 comprises melt-spun elastic material, preferably, the elastic waistband(s) 66 is made of melt-spun elastic material, more preferably, the elastic waistband(s) 66 consists essentially of melt-spun elastic material. According to an embodiment, the elastic waistband(s) 66 preferably comprises melt-spun elastane. The elastic waistband(s) 66 comprising melt-spun elastic material in form of elastic strands or elastic threads said elastic strands or elastic threads comprises, preferably consisting of, elastane and has a fineness or thickness or a linear density between 200 dtex and 1400 dtex, preferably between 400 dtex and 1200 dtex. The elastic waistband(s) 66 comprising melt-spun elastic material in form of elastic strips or flat elastics said elastic strips or flat elastics have a fineness or thickness or a linear density between 1000 dtex and 5000 dtex, preferably between 2000 dtex and 4500 dtex. The elastic waistband(s) 66 comprising melt-spun elastic material in form of elastic film said elastic film has a total basis weight comprised between 5 and 50 gsm, preferably between 8 and 30 gsm.

### Front side panels 36 and rear side panels 30

Referring to FIG. 1 and 3, as mentioned hereabove, the absorbent article 10 may have front side panels 36 and/or rear side panels 30. Only one set, or pair, of side panels may be used, preferably the rear side panels 30 with said single set of side panels comprising fasteners 32 configured to engage the landing zone or landing zone area 34. If two sets of side panels 30,36 are provided, in most instances, only one set of the side panels may have fasteners 32 to engage the landing zone 34, with the other set of side panels being free of fasteners. The side panels may be shaped in any shape such as triangular, rectangular, trapezoidal or any polygonal shape. The side panels 30,36 are discrete components joined to the backsheet 22 and/or to the topsheet 20 and/or longitudinal and/or transversal cuff(s) 40 of the absorbent article 10 either on the wearer-facing surface, on the garment-facing surface, or intermediate the two surfaces, preferably the side panels 30,36 are joined to the backsheet 22 on the garment-facing surface on the absorbent article 10. The side panels 30,36, or portions thereof, comprise an elastic material. The side panels can comprise an elastic film, elastic strands or elastics strips positioned intermediate a first nonwoven material and a second nonwoven material, said elastic film may be apertured.

The front side panels 36 and/or rear side panels 30 may comprise at least one elastically elongatable panel joined to the absorbent article 10, e.g. to the topsheet 20, backsheet 22, outer cover 26 or absorbent core 23 or ADL 25, wherein the elastically elongatable panel comprises at least one cover layer and an elastomeric film which is substantially free of N,N-dimethylacetamide and/or 1-ethylpyrrolidin-2-one attached to the cover layer. Said at least one cover layer and elastomeric film are joined by mechanical bonding, at a plurality of discrete bonding elements and wherein each said panel comprises a plurality of wrinkles having peaks and troughs formed on at least one of said cover layer. Said panel comprises an average of no more than two, preferably no more than one, wrinkles between two consecutive discrete bonding elements along a panel length extending substantially parallel to the transverse axis, and said panel comprises a wrinkle distribution of at least 1.1 wrinkles/mm, according to the method herein.

The front side panels 36 and/or rear side panels 30 may comprise at least one elastically elongatable panel joined to the chassis, wherein the elastically elongatable panel comprises at least one cover layer, and a plurality of elastic strands or strips attached to the cover layer. Said least one cover layer and elastic strands are joined by mechanical bonding, at and/or between a plurality of discrete bonding elements and wherein each said panel comprises a plurality of wrinkles having peaks and troughs formed on at least one of said cover layer. Said panel comprises an average of no more than two, preferably no more than one, wrinkles between two consecutive discrete bonding elements along a panel length extending substantially parallel to the transverse axis, and said panel comprises a wrinkle distribution of at least 1.1 wrinkles/mm, according to the method herein. The plurality of discrete bonding elements can be sinusoidally disposed are aligned such that when one or more imaginary lines are traced between bonding elements substantially sinusoidal waves are formed along the panel length, and each of the plurality of elastic strands crosses said imaginary line at a plurality of intersections, preferably at least 4 intersections, even more preferably at least 5 intersections, even more preferably from 6 to 30 intersections, even more preferably from 7 to 25 intersections, even more preferably from 8 to 20 intersections, even more preferably from 9 to 15 intersections.

Taking the above examples, the at least one elastically elongatable panel is joined to the rest of the absorbent article 10 by mechanical bonding or adhesive at a first attachment zone of said panel, and preferably wherein said panel is joined, preferably by mechanical bonding or adhesive, to a fastening member at a second attachment zone of said panel oppositely disposed from said first attachment zone at an opposite end of said panel. For example the elastic strands can be fully or partially coated with adhesive. The elastic strands may also be bonded via mechanical bonding without being coated with adhesive.

According to the present disclosure, the front and/or rear side panels 36,30 comprise elastic material which is substantially free of N,N-dimethylacetamide and/or 1-ethylpyrrolidin-2-one. For instance - taking the above examples, the elastomeric film or the elastic strands or strips are substantially free of N,N-dimethylacetamide and/or 1-ethylpyrrolidin-2-one. According to an embodiment, the front and/or rear side panels 36,30 comprise melt-spun elastic material, preferably, the front and/or rear side panels 36,30 are made of melt-spun elastic material, more preferably, front and/or rear side panels 36,30 consist essentially of melt-spun elastic material. According to an embodiment, the front and/or rear side panels 36,30 preferably comprise melt-spun elastane. The front and/or rear side panels 36,30 comprising melt-spun elastic material in form of elastic strands or elastic threads said elastic strands or elastic threads comprises, preferably consists of, elastane and has a fineness or thickness or a linear density between 200 dtex and 1400 dtex, preferably between 400 dtex and 1200 dtex. The front and/or rear side panels 36,30 comprising melt-spun elastic material in form of elastic strips or flat elastics said elastic strips or flat elastics have a fineness or thickness or a linear density between 1000 dtex and 5000 dtex, preferably between 2000 dtex and 4500 dtex. The front and/or rear side panels 36,30 comprising melt-spun elastic material in form of elastic film said elastic film has a total basis weight comprised between 5 and 50 gsm, preferably between 8 and 30 gsm.

### Elasticized edges 70

Referring to FIG. 7, the absorbent article 10 may comprise one or more pairs of elasticized edges 70 comprising one or more edge elastics 72, here a pair of elasticized edges 70 each comprising at least one edge elastics 72. These edge elastics 72 lead the elasticized edges 70 to help form a cup and thereby a seal around the legs of a wearer thanks to the elastic tension or contraction of the edge elastics 72. According to an embodiment, the absorbent article 10 comprises a topsheet 20 and a backsheet 22, the edges elastics 72 are arranged in between said topsheet 20 and a backsheet 22 within the crotch region 12. The edges elastics 72 are arranged transversally outboard of the absorbent core 23, meaning they are more proximate to the transversal edges 16,17 and the absorbent core 23 is more distal to the transversal edges 16,17. The edges elastics 72 may also be partially present in the front and rear regions 11; 13. According to the present disclosure, the edges elastics 72 comprise elastic material which is substantially free of N,N-dimethylacetamide and/or 1-ethylpyrrolidin-2-one. According to an embodiment, the edges elastics 72 comprise melt-spun elastic material, preferably, the edges elastics 72 are made of melt-spun elastic material, more preferably, the edges elastics 72 consist essentially of melt-spun elastic material. According to an embodiment, the edges elastics 72 preferably comprise melt-spun elastane. The edges elastics 72 comprising melt-spun elastic material in form of elastic strands or elastic threads said elastic strands or elastic threads comprises, preferably consists of, elastane and has a fineness or thickness or a linear density between 200 dtex and 1400 dtex, preferably between 400 dtex and 1200 dtex..

The present disclosure is not limited to one embodiment and previous embodiments can be combined. The present disclosure pertains to an absorbent article 10 comprising wherein the absorbent article 10 further comprises at least one component selected from front side panels, rear side panels, waist elastics, longitudinal and/or transversal cuffs, waist bands, gasketing elastics, barrier leg cuffs, leg elastics said at least one component comprising elastic material which is substantially free of N,N-dimethylacetamide and/or 1-ethylpyrrolidin-2-one. The absorbent article 10 can comprise two, three, four or more components comprising elastic material which is substantially free of N,N-dimethylacetamide and/or 1-ethylpyrrolidin-2-one. For example the absorbent article 10 can comprise, rear side panels 30 and waist bands 66, gasketing elastics 38 and leg elastics 41 all comprising elastic material which is substantially free of N,N-dimethylacetamide and/or 1-ethylpyrrolidin-2-one. For example the absorbent article 10 can comprise, gasketing elastics 38, leg elastics 41, waist elastics 64 all comprising elastic material which is substantially free of N,N-dimethylacetamide and/or 1-ethylpyrrolidin-2-one. The absorbent article 10 can comprise only elasticized edges 70 with edge elastics 72 comprising elastic material which is substantially free of N,N-dimethylacetamide and/or 1-ethylpyrrolidin-2-one.

As discussed hereabove, the topsheet 20 is the outermost layer of the absorbent article 10 that is in contact with the wearer's skin. The topsheet 20 may be joined to portions of the backsheet 22, the absorbent core 23, the barrier leg cuffs 40, the front and/or rear waist belt 43,42 the acquisition distribution layer 25 and/or any other layers as is known to those of ordinary skill in the art. A suitable topsheet 20 may be manufactured from a wide range of materials, such as apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may be a dual layer, spunbond nonwoven web. Each of the layers has a basis weight from about 10 gsm to about 25 gsm or 35 gsm. One layer may have similar or different hydrophilicity/ hydrophobicity profiles compared to the other layer. In another form the topsheet is an air through carded nonwoven web comprising a blend (e.g., 50-50 weight percent) of polyester fibers and bio-based polyethylene fibers. The basis weight of such a nonwoven is typically about 15 gsm, 20 gsm, 25 gsm, 35 gsm, or 40 gsm.

The backsheet 22 is generally that portion of the absorbent article 10 positioned proximate to the garment-facing surface of the absorbent core 23. The backsheet 22 may be joined to portions of the topsheet 20, the outer cover material 26, the absorbent core 23, the release paper 71 and/or any other layers of the absorbent article by any attachment methods known to those of skill in the art. The backsheet 22 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet. Backsheet films can optionally comprise bio-based materials, such as, for example, bio-based polyethylene.

The outer cover 26 material, sometimes referred to as a backsheet nonwoven, 26 may comprise one or more nonwoven materials joined to the backsheet 22 and that covers the backsheet 22. The outer cover 26 material is typically the outermost layer facing outward-i.e., towards garments or undergarments when present and away from the wearer-s skin. A caregiver interacts significantly with the outer cover 26 material when holding/comforting the wearer and/or changing the absorbent articles. The outer cover 26 material forms at least a portion of the garment-facing surface of the absorbent article 10 and effectively covers the backsheet 22 so that film is not present on the garment-facing surface. The outer cover 26 material may comprise a bond pattern, apertures, and/or three-dimensional features. The outer cover material may comprise a carded nonwoven or a multi-layered nonwoven comprising carded layers and one or more spunbond layers.

Adhesives can be employed to affix one component to another component in the article's final assembly. Adhesives can also be employed to immobilize sub-components, such as, for example, particulate matter within an absorbent core component, the different elastics elements. The adhesives in some instances are devoid of added fluorescence.

The absorbent articles 10 of the present disclosure may be placed into packages. The packages may comprise polymeric bag and an optional carton surrounding at least a portion of the polymeric bag. The polymeric bag can comprise a given amount of recyclate content such as at least 30%, preferably at least 50%, even more preferably at least 80% of recyclate content. The polymeric bag can comprise bio-based polyolefin. The optional carton can be made of fiberboard and can contain recycled material or a blend of recycled and virgin material. The package may comprise paper bag having a basis weight comprised between 30 gsm and 120 gsm. Each package may comprise a plurality of absorbent articles.

The absorbent article 10 comprises absorbent material 24 comprising cellulose fibers and/or superabsorbent polymers. Superabsorbent polymers, superabsorbent polymers particles, or SAPs refer to water-swellable, water-insoluble organic or inorganic materials capable of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm. The superabsorbent material according to the present disclosure comprises a grade of biodegradable or bipolar or biocompostable or with a bio-based content superabsorbent material. The biodegradable hydrogel-forming polymeric superabsorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose. According to some embodiment, the superabsorbent material can comprise in addition synthetic hydrogel-forming polymers. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present disclosure in an amount up to about 90% by weight.

According to an embodiment, in order to limit hazardous compounds, the superabsorbent polymers, or superabsorbent particles, are biodegradable or biocompostable or with a bio-based content and will be referenced as bioSAP hereunder. The biodegradable superabsorbent material (bioSAP) comprises a suitable biodegradable superabsorbent base material. Said biodegradable superabsorbent base material can comprise polysaccharides-derived polymers said material having crosslinked polysaccharides. Example of such crosslinked polysaccharide comprise material such as carboxymethyl cellulose based material crosslinked polycarboxy polysaccharide or carboxyalkylated polysaccharide such as carboxymethyl starch. Other example of polysaccharides include those that have a backbone substituted with ionic (polar) substituents, such as alkylcarboxylate or amine groups. Amine groups occur naturally in some polysaccharides, such as chitosan, while carboxylate groups must be artificially added to the most abundant polysaccharides, cellulose and starch, using carboxy alkyl donating reagents. The most common carboxyalkylaled polysaccharides are carboxymethyl cellulose (CMC) and carboxymethyl starch (CMS). These polysaccharides-derived polymeric material can be used as outright substitutes for conventional polyacrylates SAPs or as grafts or copolymers that may be used in conjunction with conventional polyacrylates SAPs. Said biodegradable superabsorbent base material can comprise carbohydrate fermentation-derived polymers in addition or alternatively to the polysaccharides-derived polymers. These material include components comprising alcohol such as isopentyl alcohol and 2-methyl-1-butanol, and to a lesser degree contains isobutyl alcohol, n-propyl alcohol, and small amounts of other alcohols, esters and aldehydes. In addition, n-butanol may be derived from the fermentation of acetone/ethanol or from the catalytic condensation of ethanol. The reduced or partially reduced ethers or esters containing either a hydroxyl or amine functionality are used to functionalize oxidized (acidic) starch. These functionalized starches which are carbohydrate fermentation-derived compounds can be used as outright substitutes for conventional polyacrylates SAPs or as grafts or copolymers that may be used in conjunction with conventional polyacrylates SAPs.

According to an embodiment, the core wrap substrates 34 comprise an upper core wrap 33 and a lower core wrap 34. The upper core-wrap 33 and the lower core-wrap 34 can be made of the same material such as SM (spunbond meltblown) laminate, a spunbond or a spunbond laminate e.g. SS), or a carded nonwoven. Preferably, the basis weight of the upper core-wrap 33 is greater than the basis weight of lower core-wrap 34 the basis weight of upper core wrap is comprised between 5 and 60 gsm, preferably between 15 and 50 gsm, more preferably between 18 and 45 gsm, even more preferably between 20 and 35 gsm whereas the basis weight of the bottom core wrap 34 is comprised between 5 and 15gsm, preferably between 6 and 12 gsm.

The present disclosure pertains to more environmentally friendly alternatives to conventional absorbent articles. According to an example, the absorbent article 10 according to the present disclosure comprises totally chlorine free (TCF) or elemental chlorine free (ECF) cellulose fibers. For example, these TCF or ECF cellulose fibers can comprise a Kappa number comprised between 5 and 20 as determined by any standard method such as method ISO 302:2004 or TAPPI Standard Method No. T-214-SU71, where K ≈ c*l (with K: Kappa number; c: constant ≈ 6,57 (dependent on process and wood); l: lignin content in percent). Such totally chlorine free (TCF) or elemental chlorine free (ECF) cellulose fibers are material which does not utilize chlorine in the whitening process. The absorbent article 10 may also comprise unbleached cellulose fibers or unbleached pulp. The absorbent core 23 may comprises of about 40% to 80% of biodegradable superabsorbent polymer and anywhere from 60% to 20% of the unbleached pulp, preferably between 40% and 25%. For example, some embodiments may utilize only unbleached pulp, while others may utilize unbleached pulp as a percentage of the fiber content. Specifically, in one embodiment, the unbleached pulp content of absorbent core may be as low as 25% of the fiber content and then the remaining 75% of the fiber content would be either a bleached, totally chlorine-free (TCF) or an elemental chlorine-free (ECF) pulp. Unbleached pulp, in accordance with some embodiments, may include a mixture of softwood and hardwood fiber content. Hardwood fibers generally provide much shorter and less coarse fiber with respect to softwood fibers and hence can improve capillarity. For example, in some embodiments the unbleached pulp may have as much as 60% hardwood fibers.

For example, according to the present disclosure absorbent article 10 comprises an absorbent core 23 wrapped in a 8 gsm core wrap, said absorbent core 23 comprising a mixture with biodegradable superabsorbent material comprising polysaccharides-derived polymers with a basis weight of 4.0 gsm and biodegradable superabsorbent material comprising carbohydrate fermentation-derived polymers with a basis weight of 8.8 gsm, for a total of 12.8 gsm, the absorbent core further comprising cellulosic fluff with a basis weight of 5 gsm. Another example according to the present disclosure, the absorbent article 10 comprises a topsheet made of 14 gsm polyolefin nonwoven, a backsheet comprising a laminate of a 13 gsm nonwoven and a 14 gsm impermeable film, an absorbent core wrapped in an 8 gsm core wrap comprising a polyacrylate superabsorbent material, i.e. conventional or synthetic SAP, with a basis weight of 12.3 gsm and TCF cellulose fluff with a basis weight of 7 gsm. Another example according to the present disclosure, the absorbent article 10 comprises a topsheet made of 18 gsm cotton nonwoven, a backsheet comprising a laminate of a 12 gsm nonwoven and a 15 gsm impermeable film, an absorbent core wrapped in a 12 gsm core wrap comprising biodegradable superabsorbent material comprising polysaccharides-derived polymers with a basis weight of 12.2 gsm and ECF cellulose fluff with a basis weight of 7 gsm. In each of the above example, the absorbent article 10 comprises at least one component selected from front side panels, rear side panels, waist band, gasketing elastics, barrier leg cuffs, longitudinal and/or transversal cuffs, elasticized edges, leg elastics or waist elastics comprising an elastic material which is substantially free of N,N-dimethylacetamide and/or 1-ethylpyrrolidin-2-one.

In an embodiment, the absorbent article herein further comprises an acquisition distribution layer 25 positioned between the topsheet 20 and the core wrap substrate 33,34, preferably an upper layer thereof, and wherein the majority (typically being more than 50%, preferably more than 60%, even more preferably more than 70%, even more preferably more than 80%, even more preferably more than 80%, of the surface area of said acquisition distribution layer taken from a planar view formed by the longitudinal axis y and a transverse axis perpendicular thereto) of the surface of said acquisition distribution layer is in direct contact at least with said core wrap substrate; and wherein the acquisition distribution layer comprises, preferably consists of, natural fibers. For example, the acquisition distribution layer 25 can comprise a layer consisting essentially of viscose, such as an airlaid viscose layer.

The present disclosure also pertains to a method for manufacturing an absorbent article 10 according to any of the preceding claims, comprising the following steps:
- providing a liquid permeable topsheet 20, a liquid impermeable backsheet 22, and an absorbent core 23 comprising absorbent material 24, and at least one component selected from front side panel 36, rear side panel 30, waist belt 42,43, elastic waistband 66, longitudinal and/or transversal cuffs 40, leg elastic 41, elasticized edge 70 said at least one component comprising elastic material which is substantially free of N,N-dimethylacetamide and/or 1-ethylpyrrolidin-2-one,
- joining said topsheet 20, backsheet 22 and absorbent core 23, and
- joining said at least one component comprising elastic material which is substantially free of N,N-dimethylacetamide and/or 1-ethylpyrrolidin-2-one to at least one of said topsheet 20, backsheet 22 and absorbent core 23.

The above step can be sequential or simultaneous or concomitant. For example, the absorbent core 23 can be provided on a backsheet 22, leg elastics 41 can be joined to the backsheet 22 and a topsheet 20 can be provided on the absorbent core and joined to the backsheet 22, then front and rear side panels can be joined to the backsheet 22. For example, a backsheet 22 and a topsheet 20 can be provided simultaneously to encase or envelop an absorbent core 23 and be joined together, longitudinal and/or transversal cuffs 40 can then be joined to the topsheet 20 and/or backsheet 22.

### Wrinkle distribution determination

The wrinkle distribution herein is determined by the following method.

For each side panel to be tested, the number of wrinkles is counted over a length of 10mm along the panel length (L) with the laminate in relaxed state (i.e. without applying an extension force between ends thereof, preferably taken at its "unused" state wherein "unused" herein means without the panel ever having been previously stretched since manufacture). The total number of wrinkles is then divided by 10 in order to provide the number of wrinkles per unit length. A total of 4 random locations within the elastic region (20) of the laminate may be measured accordingly and an average is calculated for each sample.

The 10mm length is typically taken from a starting position that encompasses at least one full wrinkle (i.e. a complete wrinkle comprising a peak positioned between two consecutive troughs) and by doing so if at the opposite extremity of the 10mm length only a partial wrinkle (i.e. not a complete wrinkle comprising a peak positioned between two consecutive troughs, e.g. the 10mm end at a position corresponding to the peak of a wrinkle) is formed, this is not counted as a wrinkle within the present method.

The above procedure is typically repeated for at least 4 samples of side panels and an average is calculated to provide the wrinkle distribution in wrinkles/mm as referred to herein.

### Melt-Spun process

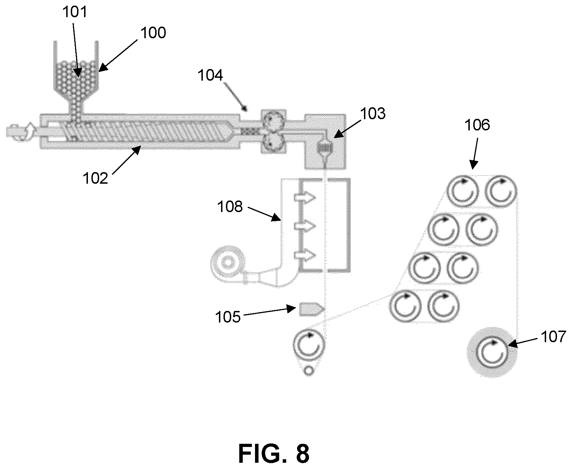

As illustrated in FIG. 8, an example of preparation of melt-spun elastic material is carried out with the following steps :
- feeding the polymeric material 101, for example elastane, in a hopper 100;
- extruding said polymeric material, by making the polymeric material flow through a screw extruder 102 where it will be melted and pushed through a spinneret 103 thereby forming filaments, the screw extruder can comprise additional elements such as a melt pump 104, a static mixer or a spin pack;
- cooling said filaments in a quenching chamber 108, the extruded filaments are quickly cooled and solidified into fine fibers, said cooling being typically achieved by exposing the fibers to a stream of cool air;
- optionally spin finishing the fibers, where the elastane fibers might undergo additional treatments, such as coating or dyeing, with a spin finish applicator 105;
- stretching fibers with a set of rollers 106 to mechanically stretched the filaments, and
- winding where the elastane fibers are wound onto a spool 107 or bobbin for storage. The winding process is continuous, with the fibers being pulled and collected at a controlled speed to prevent tangling or damage.

With the above illustrative process, it is possible to make melt-spun elastane strands that are substantially free of N,N-dimethylacetamide and/or 1-ethylpyrrolidin-2-one.

According to an embodiment, the absorbent article 10 comprises at least one component selected from front side panel 36, rear side panel 30, waist elastic 64, elastic waistband 66, gasketing elastic 38, leg elastic 41, edge elastic 72 which comprises elastic material which is substantially free of N,N-dimethylacetamide and/or 1-ethylpyrrolidin-2-one. Said elastic material comprises recycled material, preferably at least 10% of recycled material, more preferably at least 30% of recycled material, even more preferably at least 50% of recycled material. For example, the elastic material can comprise 58% reclaimed production waste. The elastic material can comprise 50% recycled polyester (PET) and 18% of plant-based material. The elastic material can also comprise bio-based elastane for example having a 30% biobased content from corn. In other words, the elastic material can comprise a recyclate content or a recycled content, the elastic material can have a post industrial or a post consumer recycled content or more simply put the elastic material can be a post-industrial recycled material or a post-consumer recycled material.

## Claims

1. Absorbent article (10) comprising:
- a liquid permeable topsheet (20),
- a liquid impermeable backsheet (22), and
- an absorbent core (23) comprising absorbent material (24) positioned between said topsheet (20) and backsheet (22),
wherein the absorbent article (10) further comprises at least one component selected from front side panel (36), rear side panel (30), waist belt (42,43), elastic waistband (66), longitudinal and/or transversal cuffs (40), leg elastic (41), elasticized edge (70) **characterized in that** at least one of said at least one component comprises elastic material which is substantially free of N,N-dimethylacetamide and/or 1-ethylpyrrolidin-2-one.

2. Absorbent article (10) according to claim 1 wherein said elastic material is selected from elastic strand, elastic film or elastic strip.

3. Absorbent article (10) according to claim 1 or 2, wherein the elastic material comprises elastane or polyester.

4. Absorbent article (10) according to any of the preceding claims, wherein the elastic material is a melt-spun material.

5. Absorbent article (10) according any of the preceding claims, wherein said at least one of said separable component is joined to the absorbent article (10) by a rubber-based adhesive or a polyolefin adhesive or by mechanical bonding.

6. Absorbent article (10) according to any of the preceding claims, wherein when said elastic material is selected from elastic strands or elastic threads said elastic strands or elastic threads have a fineness or thickness or a linear density between 300 dtex and 1500 dtex, preferably between 500 dtex and 1000 dtex, when said elastic material is selected from elastic strips or flat elastics said elastic strips or flat elastics have a fineness or thickness or a linear density between 1000 dtex and 5000 dtex, preferably between 2000 dtex and 4500 dtex, and when said elastic material is selected from elastic film said elastic film has a total basis weight comprised between 5 and 50 gsm, preferably between 8 and 30 gsm.

7. Absorbent article (10) according to any of the preceding claims, wherein the absorbent core (23) comprises biodegradable superabsorbent material (bioSAP), preferably carbohydrate fermentation-derived polymers and/or polysaccharides-derived polymers.

8. Absorbent article (10) according to any of the preceding claims, wherein the absorbent material (24) is wrapped in at least one core wrap substrate (33,34), said core wrap substrate (33,34) comprising upper and lower layers (33,34) that are joined together by one or more adhesives and/or by one or more mechanical bonds selected from the group consisting of ultrasonic bonds, thermal bonds, pressure bonds, and combinations thereof, preferably said layers are joined together at one or more attachment zones to form one or more channels (31), preferably a single channel, substantially free of absorbent material (24).

9. Absorbent article (10) according to any of the preceding claims, wherein the absorbent core (23) comprises cellulose fibers, said cellulose fibers being selected from unbleached pulp or totally chlorine free (TCF) or elemental chlorine free (ECF) cellulose fibers or any combination thereof.

10. Method for manufacturing an absorbent article (10) according to any of the preceding claims, comprising the following steps:
a. providing a liquid permeable topsheet (20), a liquid impermeable backsheet (22), and an absorbent core (23) comprising absorbent material (24), and at least one component selected from front side panel (36), rear side panel (30), waist belt (42,43), elastic waistband (66), longitudinal and/or transversal cuffs (40), leg elastic (41), elasticized edge (70) said at least one component comprising elastic material which is substantially free of N,N-dimethylacetamide and/or 1-ethylpyrrolidin-2-one,
b. joining said topsheet (20), backsheet (22) and absorbent core (23), and
c. joining said at least one component comprising elastic material which is substantially free of N,N-dimethylacetamide and/or 1-ethylpyrrolidin-2-one to at least one of said topsheet (20), backsheet (22) and absorbent core (23).

11. Method for manufacturing an absorbent article (10) according to claim 10, wherein said elastic material is selected from elastic strand, elastic film or elastic strip.

12. Method for manufacturing an absorbent article (10) according to claim 10 or 11, wherein the elastic material comprises elastane or polyester.

13. Method for manufacturing an absorbent article (10) according to claim 10 to 12, wherein the elastic material is a melt-spun material.

14. Method for manufacturing an absorbent article (10) according to claim 10 to 13, wherein said at least one of said separable component is joined to the absorbent article (10) by a rubber-based adhesive or a polyolefin adhesive or by mechanical bonding.

15. Method for manufacturing an absorbent article (10) according to claim 10 to 14, wherein when said elastic material is selected from elastic strands or elastic threads said elastic strands or elastic threads have a fineness or thickness or a linear density between 300 dtex and 1500 dtex, preferably between 500 dtex and 1000 dtex, when said elastic material is selected from elastic strips or flat elastics said elastic strips or flat elastics have a fineness or thickness or a linear density between 1000 dtex and 5000 dtex, preferably between 2000 dtex and 4500 dtex, and when said elastic material is selected from elastic film said elastic film has a total basis weight comprised between 5 and 50 gsm, preferably between 8 and 30 gsm.
